# EUROPEAN PATENT SPECIFICATION

(11) **EP 2 060 275 B1**
(45) Date of publication and mention of the grant of the patent: **12.05.2010**
(21) Application number: 07120581.9
(22) Date of filing: 13.11.2007
(51) Int. Cl.: A61L 2/07, A61L 2/26, F04C 18/344, F04C 29/02

(54) **Steam sterilizing system**
Dampfsterilisationssystem
Système de stérilisation à la vapeur

(43) Date of publication of application: 20.05.2009
(73) Proprietor: CISA S.p.A., 00040 Pomezia (Rome) (IT)
(72) Inventor: De Pian, Eros, 00040, Pomezia (Rome) (IT)
(74) Representative: Capasso, Olga

(56) References cited:
- EP-A- 1 032 432
- DE-A1- 19 830 706
- GB-A- 1 535 058

## Description

### PRIOR ART

Steam is the most economical medium for subjecting to the treatment of sterilisation most of the materials and components for which sterility is necessary. Naturally the essential condition for being able to adopt steam sterilisation is that the materials withstand high temperatures, over 100°C.

In fact steam sterilisation cycles are generally standardised to temperatures of approximately 120°C or approximately 135°C. These systems reduce the bacteria count to less than one living unit out of an initial million.

The more advanced apparatuses have software able to control and ascertain in real time that in the sterilisation chamber the conditions have been fulfilled for a predetermined time for achieving the level of sterility of the material. The parameters which determine and guarantee abatement of the bacteria count are temperature, pressure, humidity and saturation of the steam as a function of time.

The construction and performances of the apparatuses for sterilisation are governed by regulations which comprise the minimum requirements for marketing it.

The main subcomponents of the best apparatuses currently on the market are the following:
1) load structure, composed of a base frame and a possible additional module for housing the components.
2) Sterilisation chamber made in AISI 316L austenitic stainless steel or better, with interspace of approximately 70% of the surface in accordance with the European PED (Pressure Equipment Directive) 97/23/EC and marked (CE). The interspace in steam autoclaves has a dual purpose: the first is structural, allowing the sterilisation chamber to withstand high pressures with the smallest possible thickness of the walls of the chamber.

Although the ideal shape for a pressure container is spherical or cylindrical so that the internal force deforms the structure as less as possible, current regulations on steam sterilisation define the load module as a parallelepiped measuring 30x30x60 cm. It is therefore clear that a spherical or cylindrical chamber has a very high ratio of useful volume (real volume - module volume), requiring a greater quantity of steam (water + energy). Making a chamber with a parallelepiped shape the load volumes are optimised with minimum waste of space, penalising however the structural resistance. The external thicknesses of the chamber therefore have to be increased or reinforcements have to be attached.

The design solution commonly used consists of welding at a specific distance reinforcement ribbing to the outside of the chamber. In the cavities which are formed steam is fed which releases thermal energy, albeit unevenly on the internal surface of the chamber. The maximum surface covered with this solution varies from 50% to 70%.

The second purpose of the interspace is functional. By making steam circulate inside the cavities of the interspace the condensation inside the chamber reduces and drying of the load is facilitated. Steam sterilisation is based on the exchange of thermal energy of the steam with the material to be sterilised inside the chamber. When the steam enters a sterilisation chamber without external heating part of the thermal energy is used to bring the internal walls to temperature. Given that sterilisation is based on the maintaining in time of a known quantity of temperature, this constant loss of thermal energy near the walls of the chamber causes a thermal imbalance during sterilisation. The current regulations limit this imbalance to 1°C (approximately 0.7% for sterilisation at 134°C). By preheating the internal walls of the chamber, this loss of energy is reduced, improving the thermal uniformity of the internal environment, and risks of an ineffective process are avoided.

At the end of sterilisation the chamber is subjected to a negative pressure (vacuum) by means of a special pump in order to vaporise all the condensation due to the exchange of energy of the steam which has remained trapped inside the load. The vaporisation of the condensation water is generated also at a low temperature thanks to the vacuum applied and depends directly on the thermal energy accumulated by the load. Since vacuum is a thermal insulant, the thermal energy of the load is yielded to condensation which, vaporising, is extracted from the chamber. This means that the thermal energy is exhausted in a short time. The function of the preheated interspace also serves to bring new energy to the load and to the condensation in the form of thermal irradiation, improving evaporation and therefore drying of the load.

The water contained in the load at the end of the sterilisation process encourages the germination of possible micro-organisms that are still alive. Therefore it is desirable to reduce the water in the load, in order to reduce the probability of proliferation of micro-organisms. The residual humidity allowed by the regulations is +1% of the initial weight.

The more modem apparatuses are controlled entirely by electronic systems with programmable logic or by dedicated microprocessors which allow the management of cycles, the control of parameters and a check on process safety.

The phases that make up a sterilisation cycle are:
a) vacuum test
b) conditioning
c) heating
d) sterilisation
e) cooling (only for autoclaves with liquids cycle)
f) chamber discharge
g) washing
h) drying
i) aeration.

Currently the most modern and efficient systems are distinguished by the following features:
- energy consumptions amount to 14 KW/cycle of approximately 1 hour;
- consumptions of drinking water amount to 150 litres/cycle approximately;
- the machines, albeit disassembled, exceed the standard overall dimensions of the doors for access to the premises, more particularly as regards the chamber and the interspace which are inseparable, making masonry works necessary;
- the weights of all the machines require additional masonry works to support the floors;
- the need to be fed with water requires connections to the main systems of supply and discharge.

### DESCRIPTION OF THE INVENTION

There is therefore a clear advantage in the provision of apparatuses with the best performances, reduction in energy and water consumptions and smaller overall dimensions.

The authors of the present invention have developed a steam sterilisation system, more particularly an autoclave, which allows water consumption to be zeroed, without the need for connections to the water supply system, and a reduction in energy consumptions.

In a 450 litre autoclave, with current consumptions of water of approximately 150 litres/cycle, hypothesising 10 cycles a day for 260 days a year, the saving in mains water is approximately 390,000 litres and approximately 14,500 KW a year. Moreover the cycle times were also reduced by approximately 30%.

The autoclave of the invention is particularly advantageous in all those situations wherein the consumptions of water are critical and/or limiting, as for example in medical structures in poor areas, with lack or shortage of water.

The high vacuum sterilisation autoclave of the invention:
a) does not need water to obtain the vacuum;
b) has improved performances as it works in a vacuum of 1-5 mbar as opposed to the typical 30-40 mbar;
c) has a shorter total duration of the sterilisation cycle, due to simplification of the process for obtaining the vacuum;
d) has smaller size and weight and greater thermal efficiency of the interspace of the sterilisation chamber, due to the techniques of mechanical construction;
e) requires lower available electrical power (40% less), in the case of use of the new steam generator proposed;
f) does not need mains water for cooling the steam and condensation discharges.

The object of the present invention is therefore a steam steriliser comprising:
a) a treatment chamber (chamber) with an interspace for preheating and block for closure/opening for access thereto;
b) means of generating vacuum;
c) means for generating steam;
d) means for cooling the steam and condensation;
e) means for controlling the process of sterilisation;
   **characterised in that** the means of generation of the vacuum is a vacuum pump functioning in conditions of saturated or almost saturated steam at high temperatures, without a cooling system, and comprising a lubrication circuit with a tank, in which a lubricant fluid circulates that withstands high temperature and has high anti-emulsifying power, with filtration means able to separate the steam from the lubricant fluid. The pump is preferably of the rotary blade type, although the expert may be able to define other pumps, for example the lobed type.

The pump is also provided with means for the automatic disposal of possible steam residues present in the lubricant fluid, such that at each steam pumping cycle the separation of fluid/condensed steam in the tank takes place constantly.

The pump may also comprise thermostat-controlled heating means inside the tank.

The lubricant fluid to be used in the pump must have features of high specific weight, stability at high temperatures and anti-emulsifying properties.

The lubricant fluid may be mixed, in a defined proportion, with a specific additive which confers antioxidant properties to the metal surfaces of the pump. In this way the formation of rust and incrustations is avoided.

The materials and special elastomers are suitable for withstanding continuous pumping of the steam at a high temperature.

The pump is also made in a hermetic version (without oil guards) with transmission of the rotary motor/pump movement via a magnetic joint. The benefits which are obtained by the provision of this version relate to the safety and reliability of operation of the pump. In fact the risk of leakage of the fluid due to possible breakage of the oil guards is eliminated completely; moreover the operations of maintenance are restricted to only a periodical check on the level of the lubricant and to an overhaul for cleaning and replacement of the seals in the medium-long term.

In a preferred embodiment the generator of steam is of the type with low consumption.

In a preferred embodiment the means for cooling the steam and the condensation is a cooling system which does not use mains water as an exchange fluid.

### DETAILED DESCRIPTION OF THE INVENTION

The present invention will now be described in its embodiments as non-limiting examples with reference to the following drawings:
Figure 1: functional diagram of an embodiment of the steriliser.
Figure 2: functional diagram of a further embodiment of the steriliser.
Figure 3: schematic representation of the pump of the steriliser.
Figure 4: schematic representation of the steam generator according to an embodiment of the steriliser.
Figure 5: diagram of functioning of the cooling system with recovery of energy and elimination of the mains water according to an embodiment of the steriliser.
Figure 6: graph of the process phases of sterilisation.

The steam steriliser is composed of the following components:
a) a treatment space (chamber) with preheating interspace and block for closure/opening for access thereto;
b) means for generation of vacuum;
c) means for generation of steam;
d) means for cooling the steam and condensation;
e) control means.

The control system comprises a programmable controller connected to the temperature and pressure sensors, to the solenoid valves, to the contactors, to the relays, to the position sensors, to the level sensors and connected to a touch screen interface for the management of the steriliser.

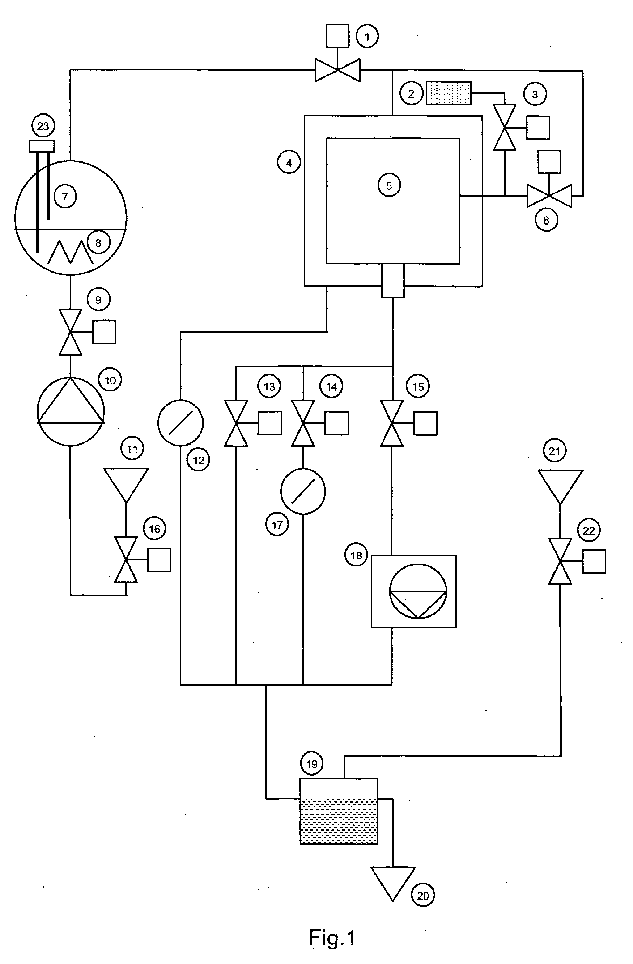

The functional diagram of the steriliser is illustrated in Figure 1. In it the numerals refer to the components according to key 1 below.

### Key 1

| Number | Description |
|---|---|
| 1 | Interspace steam valve |
| 2 | Aeration filter |
| 3 | Aeration valve |
| 4 | Interspace |
| 5 | Sterilisation chamber |
| 6 | Chamber steam valve |
| 7 | Steam generator |
| 8 | Electrical resistors |
| 9 | Generator treated water valve |
| 10 | Generator treated water feed pump |
| 11 | Treated water inlet |
| 12 | Interspace condensation discharger |
| 13 | Chamber discharge valve |
| 14 | Chamber condensation discharge valve |
| 15 | Chamber vacuum valve |
| 16 | Tank treated water feed valve |
| 17 | Chamber condensation discharger |
| 18 | Vacuum pump |
| 19 | Discharge cooling device |
| 20 | Condensation and air discharge |
| 21 | Water inlet |
| 22 | Cooling water valve |
| 23 | Generator level control |

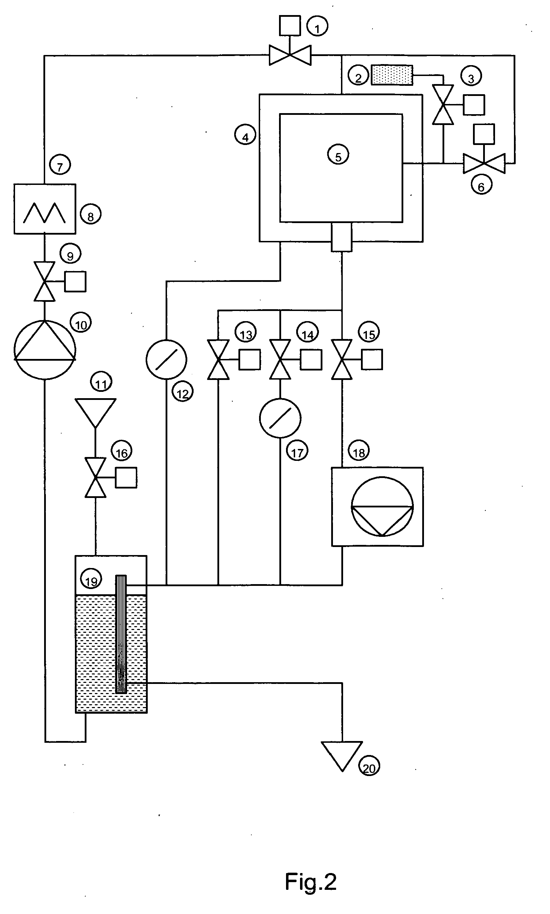

Figure 2 represents the functional diagram of a further embodiment of the steriliser, wherein the numerals refer to components in line with those of Key 1. In this embodiment the cooling system does not use mains water as exchange fluid, not therefore requiring pipes for the feeding of water and connections.

### Mechanical Construction

The general objective of the mechanical embodiment consists in the reduction in the weights and overall dimensions. Materials are therefore used which have a good mechanical strength and resistance to corrosion and which have a low specific weight, to obtain a structure able to resist a pressure of 4,500 absolute mbar, meeting all requirements of the existing directives (PED).

The reduction in the percentage of touching interspace involves various design solutions, including:
a) Connecting the ribbing with an even surface formed by a single sheet of material wherein holes are made which are later welded on the ribbing. The disadvantage of this solution is that it needs more material, with a consequent increase in weight. Therefore the solution can advantageously be made only with materials with a specific weight lower than that of the steel usually used. An example of these materials is series 6000 anodised aluminium which has properties of high resistance modulus, comparable to those of steel.
b) Producing an even surface using a single sheet of material with shapes, obtained by means of drawing. This design solution can also be produced with the same material usually used in that it requires smaller thicknesses.
c) Formation of the interspace by means of welding of a single sheet of material at the outer frame of the chamber so as not to have any point of contact between chamber and interspace.

The overall dimensions have been improved with a redistribution of the structural components. For example, for a sterilisation chamber measuring 66 cm, with a standard passage span for a door of approximately 90 cm, the remaining 12 + 12 cm represent the maximum limit of thickness of the walls of the chamber, the height of the interspace and the external thermal insulation. If a thickness of approximately 3 cm of heat-insulating lagging is hypothesised, the total height of the interspace must be approximately 8 cm.

Weights are optimised with the use of materials whose specific weight is smaller than those currently used and having an equal or improved modulus of resistance, and with a reduction in the mass of the chamber by reducing the thicknesses, although resisting the test pressures as required by the PED directive.

The block of closure/opening for access to the treatment chamber is made up of two doors in AISI 316L stainless steel connected to a counterweights balancing system and actuated by pneumatic cylinders and by two silicone seals which are dynamically thrust on the surface of the door.

The treatment space (sterilisation chamber) is made up of a parallelepiped in AISI 316L stainless steel measuring 660x660x1000 mm. (6 sterilisation modules, EN285:2006)

### Construction of a high vacuum system (RI)

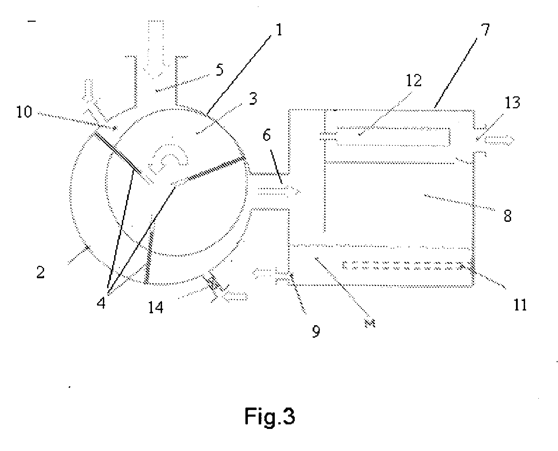

Figure 3 is a schematic representation of the pump.

Referring to Figure 3, the pump 1 is provided with a stator body 2 containing an eccentric rotor 3 with bearings to facilitate its rotation and at least one blade 4, with an aspiration conduit 5, and a discharge one 6. The pump 1 is also provided with a lubrication circuit 7, comprising a storage tank 8 with an intake point 9 of the lubricant fluid (M) placed on its base and an aspiration chamber 10. The storage tank 8 can be provided at its base with a thermostat-controlled heating resistor 11 and a coalescence filter 12, able to separate the vapours of the lubricant fluid, recovering them in the same tank. The flow of steam traverses the filter 12 together with the non-condensable gases and with them is conveyed to the outlet 13.

The pump 1 is provided with appropriate sealing means, for opening and closure of conduits, such as valves, diaphragms and oil guards.

The lubricant fluid (M) is drawn through vacuum from the intake point 9 of the tank 8 towards the aspiration chamber 10, thus ensuring a constant and optimal lubrication of the rotor 3/stator 2/blades 4 assembly and of all the other components, such as valves, diaphragms, oil guards, bearings, etc., required for the proper functioning of the pump 1.

The pump is also provided with a gas ballast device 14, placed at the base of the stator 2, for the automatic disposal of the residues of humidity present in the lubricant fluid.

The force of cohesion of the fluid (M) with the surfaces of the mechanical elements of the pump 1 (e.g. the inner walls of the stator 2) prevents the steam aspirated from the conduit 5 from scouring them, ensuring constant lubrication thereof.

The condensation in input from the aspiration conduit 5 is pumped with the air and the steam and, together with the vapours of the lubricant fluid (M), is conveyed into the storage tank 8. The high anti-emulsifying power of the lubricant fluid, its high temperature and the difference in specific weight with the water, encourage the fluid/condensation separation and the localisation of the latter at the top of the tank 8. Thus constant dehydration of the lubricant fluid (M) in the lower layers of the tank 8 is ensured. The fluid is drawn by means of the intake point 9, due to a difference in pressure, inside the aspiration chamber 10 of the pump 1.

In this way the pump constantly succeeds, even after having performed repeated cycles of pumping of the steam, in reaching a high degree of vacuum in the aspiration conduit 5.

The device 14 known as "gas ballast" allows automatic disposal of the residues of humidity present in the lubricant fluid (M). The feeding of air via the device 14 reduces the partial pressure of the condensable gases present in the mixture during compression. In this way their transformation into the liquid phases is avoided, facilitating their expulsion and preventing them from mixing with the lubricant fluid.

An effective coalescence filter 12, placed at the discharge, separates the vapours of the lubricant fluid, recovering them in the bath of the tank. The flow of steam traverses the filter together with the non-condensable gases and with them is conveyed to the outlet 13. Any condensation that remains inside the tank is arranged floating on the lubricant liquid in the tank from where, due to the high temperature of the fluid, it is re-circulated in the form of steam and disposed through the outlet 13.

The efficacy of the filter reduces to negligible values the consumption of lubricant fluid used, contributing to the safeguarding of the surrounding environment.

To further guarantee functioning also in the cases of pumping of quantities of steam above the limits of thermal balance of the pump, a thermostat-controlled heating resistor 11 was inserted in the fluid storage tank 8. In this way the latent heat of evaporation is supplied, necessary for disposal of the excess condensation present on the bath.

The seals, diaphragms, blades, oil guards, bearings, etc. are made with materials resistant to temperatures of continuous operation of at least 130°C.

In fact the body of the pump functions without the forced cooling system, obtaining an increase of approximately 30°C of the temperature value under normal conditions.

Cooling, in traditional pumps lubricated with mineral oil, serves to keep the working temperature low so as to avoid overheating and consequent loss of the lubricating power of the oil. The pump of the invention has instead to work at high temperatures, above 100°C, to prevent condensation of the aspirated steam and to reduce to a minimum the presence of residual humidity in the lubricant fluid (M).

The lubricant fluid used, Fomblin (Solvay Solexis), has high specific weight, more than double that of mineral oil, and has a very high chemical stability at high temperatures, with outstanding anti-emulsifying and antioxidant properties.

The lubricant fluid (M) can be mixed in a defined proportion, with a specific additive which confers antioxidant properties to the metal surfaces of the pump. In this way the formation of rust and encrustations is avoided.

The pump 1 is also made in a hermetic version (without oil guards) with transmission of the rotary motor/pump movement via a magnetic joint. The benefits obtained from the making of this version relate to safety and the reliability of operation of the pump. In fact the risk of leakage of the fluid due to a possible breakage of the oil guards is eliminated in full; moreover the operations of maintenance are limited to only a periodical check on the level of the lubricant and an overhaul for cleaning and replacement of the seals in the medium-long term.

### Embodiment of a generator of steam with low consumption (SP)

The embodiment of a steam generator with low consumption involves the use of materials with high heat conductivity (for example aluminium, for the body of the generator) and an outstanding resistance to corrosion (for example steel, for the elements for heating of the water), and also the construction of a structure with high thermal inertia.

Traditional electric steam generators are composed of a cylindrical container in steel wherein treated water is fed by means of an external pump, and later heated by immersed electric resistors until it evaporates. The level of the water inside the generator is controlled by means of mechanical float level sensors or by electronic conductivity control. The exchange thermal energy between the electric resistors and the water is due to the contact surface of the heating elements. On average a steam generator installed for the operation of a 450 litre steriliser has a power which varies from 27 KW up to 45KW and has a volume of approximately 75 litres. A generator has been developed which is able to transfer as fast as possible the thermal energy of the elements for heating the water by means of a single body with high thermal inertia.

It is made in a single block of material with high thermal inertia which is small in size, about 30x30x30 cm., wherein the heating resistors are embedded and a passage formed for the feed water and the relative steam outlet. The generator does not therefore need regulation of level in that the heat exchange takes place instantaneously.

For the body of the generator, materials such as series 6000 aluminium or copper or other materials featuring high heat conductivity can advantageously be used.

In the body made in this way the heating resistors and a coil for feeding water and constant removal of steam are inserted, as well as a system for the control of the temperature. For this type of generator a level control system is not required in that the water fed into the inside by means of a pump is vaporised instantaneously. The temperature of regulation of the body is established after a series of working tests on an optimal value for the correct and total evaporation of the water fed.

The heating elements are made together with the body, with a scalar modular system. In this way it is possible to install a proportionally correct power also for sterilisers of different dimensions.

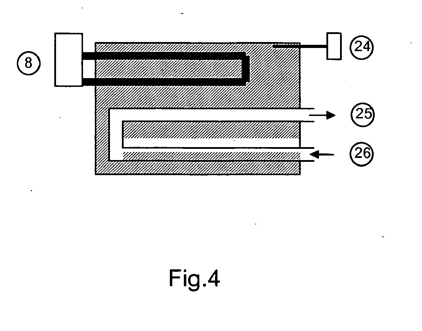

Figure 4 is a schematic representation of the steam generator.

In it the numbers refer to the components according to Key 2 below.

### Key 2

| Number | Description |
|---|---|
| 8 | Electric resistors |
| 24 | Temperature probe |
| 25 | Steam outlet |
| 26 | Water inlet |

### System of cooling of condensation and steam (SP)

The cooling system does not use mains water as an exchange fluid.

The system for cooling the discharge effluent used is usually made up of a container wherein the "hot" discharges (steam at 143°C and condensation at 100°C) is conveyed, cooled by means of water from the mains to a temperature lower than 60°C. These devices serve to avoid costly building work to produce discharges resistant to high temperatures, over 60°C.

The cooling system developed avoids the use of mains water as exchange fluid. The heat energy of the discharges is recovered and is conveyed in the treated water which is fed into the generator and later vaporised. This system allows a lower consumption of electrical energy by the steam generator, since it uses water which is already preheated.

Different systems are produced, including:
1) Use of the air as a system of cooling via a system of forced ventilation and an exchange radiator.
2) Use of electrical energy as cooling, via a system of cryopumping (chiller) with closed circuit. This type of cooling involves the design of the pumping system composed of a compressor and heat exchange elements.
3) Use of electrical energy as cooling via Peltier cells. By applying the Peltier cells to the system thermal energy is removed using electrical energy. Counter-indications in this system include the use of high electrical powers.
4) Use of compressed air as a system of cooling. This technology exploits the expansion of the gas and its relative removal of thermal energy.

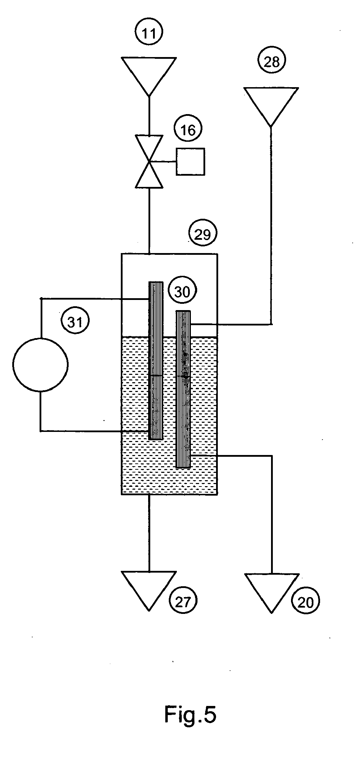

The system of cooling with energy recovery and elimination of the mains water functions according to the diagram illustrated in Fig. 5. In it the numbers refer to the components according to Key 3 below.

### Key 3

| Number | Description |
|---|---|
| 11 | Treated water inlet |
| 28 | Discharge condensation and steam inlet |
| 16 | Valve for feeding treated water |
| 31 | Cooling system |
| 27 | Removal of hot treated water for the steam generator |
| 20 | Free discharge < 60°C |
| 30 | Heat exchange elements |
| 29 | Tank |

The discharge condensation and steam bring the temperature of the treated water in the tank to approximately 60°C. When this threshold is exceeded, the system of cooling lowers the temperature by means of the exchange elements. The system of cooling can also be made via a compromise between the various systems mentioned, with the primary objective of avoiding the need for adding discharges of any kind in the atmosphere or network. For example a system of cryopumping has been produced, combined with a system of cooling with air of reduced unit.

### Process of sterilisation

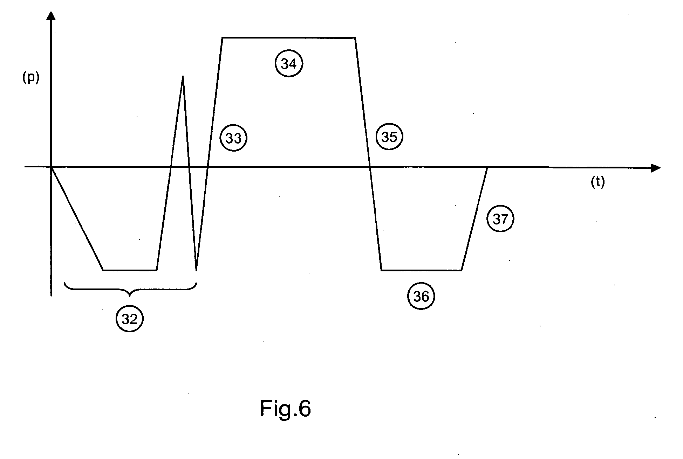

The process of sterilisation is composed of the following phases and illustrated in the graph in Figure 6:
1) conditioning
2) heating
3) sterilisation
4) chamber discharge
5) drying
6) aeration.

The numbers shown in Figure 6 refer to the various phases of the process of sterilisation according to the Key 4 below.

### Key 4

| Number | Description |
|---|---|
| 32 | Conditioning |
| 33 | Heating |
| 34 | Sterilisation |
| 35 | Chamber discharge |
| 36 | Drying |
| 37 | Aeration |

### 1) Conditioning

This phase serves to remove the air inside the sterilisation chamber and from the material, to ensure the presence of saturated steam during sterilisation. The air is removed by the vacuum pump described above; the lower the pressure reached during this phase, the better the heat distribution during sterilisation. The autoclave is able to reach conditions of high vacuum and therefore the sequence of pulsations of steam/vacuum typical of the conditioning phase of the traditional sterilisation autoclaves (not with high vacuum) is no longer necessary. The steam/vacuum pulsations have been necessary to date because they encourage the extraction of the air and the penetration of steam inside the masses to be sterilised, as compensation of the insufficient degree of vacuum that can be obtained to date. As a result this phase of the process is faster, with a saving in the times of the entire sterilisation cycle.

### 2) Heating

This phase brings the temperature of the chamber, and the load inside it, to the set one established for sterilisation.

### 3) Sterilisation

In this phase the material is maintained at the temperature established for the time necessary for abatement of the bacteria count.

### 4) Chamber discharge

In this phase the steam is removed from the chamber up to atmospheric pressure.

### 5) Drying

In this phase a vacuum is maintained for a preset time inside the chamber to allow evaporation of the condensation from the load.

### 6) Aeration

In this phase sterile air is fed to balance the pressure in the chamber for subsequent opening.

The sequence of these phases controlled by a PLC (Programmable Logic Controller), allows an ideal sterilisation cycle to be obtained with a lower consumption of electrical energy, virtually zero consumption of water for the cooling and the vacuum, and in a shorter time.

### Analysis

The following is a description of a typical sterilisation process performed on a traditional sterilisation autoclave equipped with a liquid loop pump.

The various phases of the process are:
a) Loading of the chamber of the autoclave with the objects to be sterilised.
b) Emptying of the chamber by means of a traditional liquid loop pump.
c) Pulsations of steam/vacuum (2 to 3) in the chamber. The pulsations encourage the removal of the air and the penetration of the steam inside the masses to be sterilised, to compensate the insufficient degree of vacuum of the liquid loop pump whose residual pressure is >30 mbar (limit vacuum).
d) Feeding into the chamber of saturated steam at 3 bar at the temperature of 134°C.
e) Steam sterilisation phase (approximately 10 min).
f) Release of the steam from the chamber towards atmospheric pressure.
g) Extraction via a vacuum pump of the residual saturated steam in the chamber.
h) Subsequent phase of drying in a vacuum of the sterilised objects.
i) Feeding of sterile air into the chamber.
j) Opening of chamber and removal of sterilised objects.

Total time of sterilisation cycle: approximately 45 min.

With an autoclave of the invention, it was possible to make the following improvements, applicable to the following phases of the process described:
a) Emptying of the chamber by means of a pump with high degree of vacuum without the use of water.
b) Elimination or reduction of the pulsations of steam, possible only with the use of a pump with a high degree of vacuum.
c) Extraction of the residual steam via a pump with a high degree of vacuum.
d) Forced drying of the sterilised objects by means of a pump with a high degree of vacuum able to reach residual pressures better than 2 mbar.

Total time of the sterilisation cycle: approximately 32 min.

## Claims

1. Steam steriliser comprising:
a) a treatment chamber, with preheating interspace and block for closure/opening for access thereto;
b) means for generating the vacuum;
c) means for generating steam;
d) means for cooling the steam and condensation;
e) means for controlling the sterilisation process,
**characterised in that** the means for generation of the vacuum is a vacuum pump functioning in conditions of saturated or almost saturated steam at high temperatures
without a cooling system, and comprising a circuit of lubrication with a tank, wherein a lubricant fluid circulates that is resistant to high temperatures and with a high anti-emulsifier power, with means of filtration able to separate the steam from the lubricant fluid.

2. Steam steriliser according to claim 1, wherein the pump is of the rotary blade type.

3. Steam steriliser according to claim 1 or 2, wherein the pump is provided with means for automatic disposal of any residues of steam present in the lubricator fluid, such that at each cycle of pumping of the steam the separation of fluid/condensed steam in the tank takes place constantly.

4. Steam steriliser according to one of the previous claims, wherein the pump comprises thermostat-controlled heating means inside the tank.

5. Steam steriliser according to one of the previous claims; wherein the steam generator is of the low consumption type.

6. Steam steriliser according to one of the previous claims, wherein the means for cooling the steam and condensation is a cooling system which does not use mains water as exchange fluid.

## Patentansprüche

1. Dampfsterilisator, der Folgendes umfasst:
a) eine Behandlungskammer, mit einem Vorwärmzwischenraum und einer Blockierung für den Verschluss/die Öffnung für einen Zugang zu derselben,
b) Mittel zum Erzeugen des Vakuums,
c) Mittel zum Erzeugen von Dampf,
d) Mittel zum Kühlen des Dampfes und zur Kondensation,
e) Mittel zum Steuern des Sterilisationsvorgangs,
**dadurch gekennzeichnet, dass** das Mittel zum Erzeugen des Vakuums eine Vakuumpumpe ist, die unter Bedingungen von gesättigtem oder nahezu gesättigtem Dampf bei hohen Temperaturen ohne eine Kühlsystem arbeitet, und einen Schmierkreis mit einem Tank umfasst, worin ein Schmierfluid umläuft, das gegenüber hohen Temperaturen beständig ist und eine hohe Antiemulsionskraft hat, mit Filtrationsmitteln, die dazu in der Lage sind, den Dampf von dem Schmierfluid abzuscheiden.

2. Dampfsterilisator nach Anspruch 1, wobei die Pumpe vom Rotationsschaufeltyp ist.

3. Dampfsterilisator nach Anspruch 1 oder 2, wobei die Pumpe mit Mitteln zum selbsttätigen Beseitigen jeglicher in dem Schmierfluid vorhandener Dampfreste versehen ist derart, dass bei jedem Pumpzyklus des Dampfes stetig das Abscheiden von Fluid/kondensiertem Dampf in dem Tank stattfindet.

4. Dampfsterilisator nach einem der vorhergehenden Ansprüche, wobei die Pumpe durch Thermostat gesteuerte Heizmittel innerhalb des Tanks umfasst.

5. Dampfsterilisator nach einem der vorhergehenden Ansprüche, wobei der Dampferzeuger von dem Typ mit niedrigem Verbrauch ist.

6. Dampfsterilisator nach einem der vorhergehenden Ansprüche, wobei das Mittel zum Kühlen des Dampfes und zur Kondensation ein Kühlsystem ist, das nicht Leitungswasser als Austauschfluid verwendet.

## Revendications

1. Stérilisateur à vapeur comprenant :
a) une chambre de traitement, avec un espace de préchauffage et un bloc pour la fermeture/l'ouverture de l'accès à celle-ci ;
b) un moyen de génération de vide ;
c) un moyen de génération de vapeur ;
d) un moyen de refroidissement de vapeur et de condensation ;
e) un moyen de contrôle du procédé de stérilisation,
**caractérisé en ce que** le moyen de génération de vide est une pompe à vide fonctionnant dans des conditions de vapeur saturée ou presque saturée, à des températures élevées, sans système de refroidissement, et comprenant un circuit de lubrification avec un réservoir, dans lequel circule un fluide lubrifiant résistant aux températures élevées et avec une forte puissance d'anti-émulsifiant, avec un moyen de filtration capable de séparer la vapeur du fluide lubrifiant.

2. Stérilisateur à vapeur selon la revendication 1, dans lequel la pompe est du type à pale rotative.

3. Stérilisateur à vapeur selon l'une des revendications 1 ou 2, dans laquelle la pompe est pourvue d'un moyen d'évacuation automatique de tous les résidus de vapeur présents dans le fluide lubrifiant, de sorte qu'à chaque cycle de pompage de vapeur, la séparation entre le fluide et la vapeur condensée dans le réservoir a lieu de façon constante.

4. Stérilisateur à vapeur selon l'une des revendications précédentes, dans lequel la pompe comprend un moyen de chauffage contrôlé par thermostat à l'intérieur du réservoir.

5. Stérilisateur à vapeur selon l'une des revendications précédentes, dans lequel le générateur de vapeur est du type à faible consommation.

6. Stérilisateur à vapeur selon l'une des revendications précédentes, dans lequel le moyen de refroidissement de la vapeur et de la condensation est un système de refroidissement n'utilisant pas l'eau du robinet comme fluide d'échange.
